# EUROPEAN PATENT APPLICATION

(11) **EP 2 088 191 A1**
(43) Date of publication of application: **12.08.2009**
(21) Application number: 08002362.5
(22) Date of filing: 08.02.2008
(51) Int. Cl.: C12N 5/06, C12N 15/87

(54) **Induced blastocyst-like structures, methods of production and uses of the same**

(71) Applicant: Rheinische Friedrich-Wilhelms-Universität Bonn, 53113 Bonn (DE)
(72) Inventor: Schorle, Hubert, 53125 Bonn (DE); Woynecki, Tatiana, 53129 Bonn (DE); Egert, Angela, 53639 Königswinter (DE); Buhl, Sandra, 53115 Bonn (DE)
(74) Representative: Lahrtz, Fritz

(57) **Abstract**

The present invention relates to a method of producing an induced blastocyst-like structure; an induced blastocyst-like structure and uses thereof; a method of producing a non-human embryo or non-human animal; a kit suitable for the production of an induced blastocyst-like structure, a non-human embryo or non-human animal; and the use of a modified embryonic stem cell for the production of an induced blastocyst-like structure, a non-human embryo or a non-human animal.

SEQUENCE LISTING

<110> Rheinische Friedrich-Wilhelms-Universität Bonn

<120> Induced blastocyst-like structures, methods of production and
uses of the same

<130> P65408EP

<160> 2

<170> PatentIn version 3.4

<210> 1
<211> 24
<212> DNA
<213> Artificial

<220>
<223> Primer

<400> 1
ggatcccgaa ttgaaatggg tgct 24


<210> 2
<211> 27
<212> DNA
<213> Artificial

<220>
<223> Primer

<400> 2
ggatcctcag atcgtgttgg ggaagcc 27

## Description

The present invention relates to a method of producing an induced blastocyst-like structure; an induced blastocyst-like structure and uses thereof; a method of producing a non-human embryo or non-human animal; a kit suitable for the production of an induced blastocyst-like structure, a non-human embryo or non-human animal; and the use of a modified embryonic stem cell for the production of an induced blastocyst-like structure, a non-human embryo or a non-human animal.

Embryonic stem (ES) cells, particularly mammalian embryonic stem cells, are an important tool, especially in the field of molecular genetics and stem cell research. These cells may be used to generate animals such as mice; however, the cells have to be injected into previously obtained blastocysts. This step requires complex facilities, which have to be operated by highly trained personnel. In order to obtain a blastocyst, ovulation is induced by hormones. Thereafter, the donor animals are killed in order to take the blastocysts from the animals. Altogether, the method is logistically complex, animal consuming and expensive on the one hand, but on the other hand it has become an integral part of today's work at universities as well as in industrial applications.

The above method has to be carried out as most of the embryonic stem cells obtainable for research or scientific processes are pluripotent, but not totipotent. Pluripotent embryonic stem cells are capable of differentiating into all derivatives of the three primary germ layers: ectoderm, endoderm and mesoderm. These include each of the more than 220 cell types of an adult body. Pluripotency distinguishes embryonic stem cells form multipotent progenitor cells found in the adult; these only form a limited number of cell types. Totipotency is the ability of a single cell to divide and produce all the differentiated cells in an organism including extra embryonic tissues. Particularly with respect to the blastocyst pluripotent embryonic stem cells are capable of building the inner cell mass (embryoblast), but not the cells of the trophectoderm (trophoblast) surrounding the inner cell mass and the blastocyst cavity (blastocoele). The blastocyst is a structure formed in early embryo genesis, after the formation of the blastocoele, but before implantation. As detailed above, it possesses an inner cell mass or embryoblast, which subsequently forms the embryo, and an outer cell mass or trophoblast, which forms the placenta. Usually, the human blastocyst comprises 50-150 cells. In the natural animal development the blastocyst is preceded by a zygote and succeeded by an embryo.

In accordance with the problems detailed above, it was an object of the present invention to avoid the above procedure and to provide an alternative method of producing a multicellular structure replacing the above described blastocyst.

Surprisingly, we have found that such a multicellular structure replacing a natural blastocyst may be produced by an alternative method. For this, an embryonic stem cell line has been genetically modified to obtain a transgene, particularly an inducible transgene, whose induction causes ES cells to differentiate into cells of the trophectoderm cells (ES^{TSi}). These ES^{TSi} cells have been incubated with ES cells of interest (these cells are intended to generate the animal in question). When co-culturing both types of ES cells, the formation of a blastocyst-like structure can be observed. These structures have been referred to as induced blastocyst-like structures or iBLASTs. The iBLASTs were analyzed and showed expression of the known marker genes, particularly cdx2 for trophectoderm cells and Oct4 for cells for the inner cell mass. These iBLASTS were transferred into the uteri of pseudo pregnant females. We were able to show that embryos were formed out of these iBLASTS.

Accordingly, in a first aspect the present invention relates to a method of producing an induced blastocyst-like structure comprising the step of:
a) co-cultivating a first population of embryonic stem cells modified to allow for differentiation into trophectoderm with a second population of embryonic stem cells under conditions conducive to the production of an induced blastocyst-like structure thereby producing an induced blastocyst-like structure.

The above method provides the following advantages and provides new opportunities:
- It may be carried out easily and does not require complex injection devices.
- It may be applied in any scale, including laboratory scale, pilot plant scale and industrial scale, since an almost unlimited number of iBLASTs may be generated.
- It is an alternative to chimera production by blastocyst injection or morula aggregation. The approach saves lab-animals' lifes, since there is no need in producing blastocysts or morulae from livestock.
- It saves time, since the animals produced are 100% derived from the input ES cells instead of being chimeric and since the animal can be analyzed directly saving one generation of backcrossing (i.e. 3 months).
- In addition, there is no need to superovulate donors and follow time-consuming mating schemes. Also the cost and labor-intensive part of blastocyst injection becomes obsolete, which saves on equipment and manpower.
- It is of great value for all institutions dealing with the generation of transgenic animals, in particular transgenic mice, for the analysis of gene function, pharmaceutical effects and/or stem cells.
- It may be used for any mammalian species; particularly breeding farms in the field of agriculture may use this method in order to obtain hybrid races by cloning (these animals can be obtained by co-cultivation of ES^{TSi} together with ES cells or induced pluripotent stem (iPS) cells obtained from these animals).
- It could be used in order to rescue endangered animals (for this, genetically manipulated ES^{TSi} cells or of a near related species could be generated and a transspecies iBLAST could be produced by mixing ES cells or iPS cells of the species in question with the first cells which could be transferred into the uterus).
- Having shown that iBLAST can be produced which give rise to offspring in mice, this technique can be easily adapted to work in other species as well. Foremost livestock (farm animals such as cow, sheep and pig) where it is often necessary to keep a very specific hybrid gene pool may profit from this technique. ES cells generated from these hybrids (via culturing blastocysts or reprogramming and generating iPS cells) could, in conjunction with species specific ES^{TSi} cells help in generation of clonal offspring.
- Another application would be the production of pig for meat production, where it is highly appreciated to avoid male offspring. Currently the breeders follow the 'herodes principle' meaning that all male offspring (i.e. 50%) are killed. Here, the production of iBLASts using female ES cells would abolish this problem and maximize the breeding output.
- At last, ES cells generated from species which face extinction could be brought together with ES^{TSi} cells from closely related species enabling cross-fostering.

As detailed above, in a first aspect the present invention relates to a method of producing an induced blastocyst-like structure comprising the step of:
a) co-cultivating a first population of embryonic stem cells modified to allow for differentiation into trophectoderm with a second population of embryonic stem cells under conditions conducive to the production of an induced blastocyst-like structure thereby producing an induced blastocyst-like structure.

An induced blastocyst-like structure relates to a blastocyst-like structure that may be obtained by co-cultivating two distinct populations of embryonic stem cells (as defined herein). The blastocyst-like structure resembles a blastocyst in that it possesses an inner cell mass (embryoblast), which subsequently forms the embryo, and an outer cell mass (trophoblast), which forms the extra embryonic tissues such as placenta. Our analyses show that the iBLAST organizes itself like the blastocyst. It shows cavitation (blastocoele-like structure). Also expression of cdx-2 can be seen in the cells surrounding the cavity, while nests of cells positive for Oct3/4 attached to the cdx-2 positive cells facing the blastocoel are detectable. The overall morphology and size pretty much resembles the blastocyst (without the zona pellucida).

Like a blastocyst the structure is capable of developing into an embryo including extra embryonic tissue if maintained under suitable conditions (wherein extra embryonic tissue and the embryo are formed by the first and second population of embryonic stem cells, respectively). For example, the structure may be transferred into a recipient animal to induce pregnancy into the recipient and develop into a normal embryo or even animal.

The first population of embryonic stem cells is an embryonic stem cell as defined below in the context of the stem cells of the second population, but modified to allow for differentiation into trophectoderm. As detailed above, available stem cells or stem cell lines are usually pluripotent, but not totipotent, and, therefore, not capable of developing into extra embryonic tissues. Accordingly, the first population of cells is modified to overcome this drawback.

This may be accomplished by genetically modifying the embryonic stem cells of the first population. Genetically modifying relates to any known method of genetic engineering, recombinant DNA technology, genetic modification/manipulation and gene splicing and involves direct manipulation of a cell's or organism's genes. Genetic modification uses the techniques of molecular cloning and transformation. There is a number of ways through which genetic engineering is accomplished as well known to the person skilled in the art.

Essentially, the process has four main steps.1) Isolation or identification of the gene of interest, 2) Insertion of the gene into e.g. a vector, 3) Transformation of cells and optionally 4) Tests to isolate genetically modified cell or organism.

Isolation is achieved by identifying the gene of interest that the scientist wishes to insert into the organism, usually using existing knowledge of the various functions of genes. DNA information can be obtained from cDNA or gDNA libraries, and amplified using PCR techniques. If necessary or appropriate, further modification may be carried out ligating alternative or additional regulatory elements.

Insertion of a gene into a vector such as a plasmid can be done once the gene of interest is isolated. Other vectors can also be used, such as viral vectors, and non-prokaryotic ones such as liposomes, or even direct insertion using gene guns. Restriction enzymes and ligases are of commonly used for inserting or removing DNA sequences. The vector is used to transform the target cell.

After transformation, cells of organism to be modified can be isolated from those that have not been modified in various ways. This includes methods involving resistance to certain chemicals (such as antibiotics) as known to the skilled person.

In a preferred embodiment the cells of the first population are genetically modified to include a gene that provides for differentiation of the cells into trophectoderm. Examples of these genes include a caudal related homeobox-2 (cdx-2) gene, a transcription factor activating enhancer binding protein 2 gamma (TFAP2g) gene, Eomeosdermin (Eomes) and/or a TEA domain family member 4 (TEAD4) gene, particularly a cdx-2 gene.

The genes have been shown to be important in trophectoderm specification (for AP-2 factors own unpublished results). Deletion of the genes is early embryonic lethal affecting the formation and/or survival of trophectoderm cells. Moreover, expression of cdx-2, Eomes and AP-2gamma in ES cells results in trophectoderm induction and subsequent differentiation of ES Cells in TS cells.

Caudal type homeobox transcription factor 2, also known as cdx2, is a gene that directs early embryogenesis. It is required to form the placenta. Surprisingly, it has been found that the induced expression of cdx2 in a subpopulation of stem cells allows for a method of producing an iBLAST (see Example).

While the above mentioned genes are required 'in vivo' to form trophectoderm, it is envisioned that proteins with analogous functional elements (i.e. family members within the cdx, AP-2 and TEAD family of proteins) are able to functionally replace the above mentioned genes. Accordingly, other members of the family members within the cdx, AP-2 and TEAD family of proteins may be used as well in order to provide a first population of embryonic stem cells modified to allow for differentiation into trophectoderm

Preferably, the expression of the gene(s) used for the modification of the first population of stem cells is inducible; however, the gene(s) may be also constitutive expressed. Examples of inducible expression include the use of
- an inducible promoter, wherein the gene is under the control of an inducible promoter (Examples of inducible receptors include tetracycline responsive promoters, e.g. the Tet On or Tet off system (Bujard, ZMBH Heidelberg; activator/ligand tetracycline), Tet Operator-containing promoters, interferon-inducible promoters (activator/ligand interferon) and heavy metal-inducible promoters (activator heavy metals) or
- a controllable ligand binding domain of a nuclear hormone receptor (LBD) or a functionally active variant thereof, particularly a domain of the estrogen receptor or variant thereof, wherein the gene is under the control of a controllable ligand binding domain of a nuclear hormone receptor (LBD) or a functionally active variant thereof (Examples of a include nuclear hormone receptor (LBD) or a functionally active variant thereof ER^{T} (see, below; ligand tamoxifen) and glucocorticoid receptor (ligand dexamethasone)).

It is believed that the heat shock proteins that are bound to the ligand binding domain of a nuclear hormone receptor (LBD) and that dissociate upon ligand binding keep the controlled gene inactive in the absence of ligand (Picard, Curr Opin Biotechnol, 5, 511-515 (1994)). This inhibition could occur either by sterical hindrance or by partial unfolding of the protein structure. In the context of the present invention a LBD of any nuclear hormone receptor may be used. Nuclear hormones receptors are a class of protein molecules found within the interior of cells that are responsible for sensing the presence of hormones and certain other molecules. Nuclear receptors have the ability to directly bind to DNA and regulate the expression of adjacent genes. Nuclear receptors normally are only active in the presence of ligand. Examples of nuclear hormone receptors of the estrogen-like receptor family, which is preferred, include without limitation estrogen receptor (estrogen receptor-a, estrogen receptor-β), an estrogen related receptor, a ketosteroid receptor such as the glucocorticoid receptor, the mineralocorticoid receptor, the progesterone receptor or the androgen receptor. Alternative receptor subfamilies are thyroid hormone receptor-like, retinoid X receptor-like, nerve growth factor IB-like, steroidogenic factor-like and germ cell nuclear factor-like receptors.

Regulated fusion proteins have particularly been constructed with the LBD of the well characterised estrogen receptor (ER), either in its wildtype form that responds to the natural ligand estradiol or by the use of receptor mutants that can be activated by synthetic ligands like 4-OH-tamoxifen. Examples of variants include a murine ER LBD that contains a single point mutation replacing a glycine by an arginine residue at position 525 of the mouse or the analogous position 521 of the human estrogen receptor (ER^{T}) (Brocard, et al., Proc Natl Acad Sci U S A, 94, 14559-14563 (1997)) (Danielian, et al., Mol Endocrinol, 7, 232-240 (1993)) and a human ER LBD mutant harbouring three point mutations (G400V/M543A/L544A, named ER^{T2}) described by Feil (Feil, et al., Biochem Biophys Res Commun, 237, 752-757 (1997)). A particularly suitable and preferred controllable domain of a nuclear receptor is described in the Example.

As detailed for the estrogen receptor, a variant of the LBD may be used, wherein the variant is functionally active. Functional active variants are obtainable by changing the sequence of the LBD and are characterized by having a biological activity similar to that displayed by the LBD from which it is derived, particularly including the ability to be activated upon binding of a ligand. Ability to be activated can be determined by comparing ligand-induced expression mediated by LBD and variant thereof. The variant of a LBD is functionally active in the context of the present invention, if the activity of the fragment amounts to at least 50 %, preferably at least 75 %, more preferably at least 80 %, even more preferably at least 85 %, still more preferably at least 90 %, particularly at least 95 %, most preferably at least 99 % of the activity of the LBD without sequence alteration.

In general, the expression of the gene(s) under the control of an inducible promoter or controllable ligand binding domain of a nuclear hormone receptor (LBD) or a functionally active variant thereof is induced by the addition of a chemical compound, wherein the compound is to be chosen depending on the promoter/receptor. Examples of suitable combinations are given above.

Particularly preferred is the use of 4-hydroxy-tamoxifen in combination with the ligand binding domain of estrogen receptor ER^{T} (see above) or variants thereof, especially in combination with the construct as detailed in the Example. The expression of the gene(s) may be induced during and/or before co-cultivation of the embryonic stem cells of the first and second population. As detailed above, the expression is in general induced by addition of a suitable chemical compound (depending on the prevailing promoter or ligand binding domain) in an effective concentration. If a ligand binding domain of an estrogen receptor is used, the stem cells may be incubated with 4-hydroxy-tamoxifen, e.g. in a concentration of about 1 µg/µl.

The second population of cells is any population of embryonic stem cells, especially a pluripotent embryonic stem cell. Embryonic stem cells (ES cells) are stem cells derived in general from the inner cell mass of a blastocyst. Human embryos reach the blastocyst stage 4-5 days post fertilization, at which time they consist of 50-150 cells. After removal the cells may be maintained and propagated in cell culture. Under suitable conditions the cells remain in their undifferentiated state, unless growth factors are added in order to induce differentiation.

Embryonic stem cells (ES cells) were first derived from mouse embryos in 1981 by Martin Evans and Matthew Kaufman and independently by Gail R. Martin. Today, a great number of embryonic stem cells and embryonic stem cell lines is available, including those from humans. Many of these may be used in the context of the present invention. However, those described in the Example are preferred.

Also encompassed by the term embryonic stem cells in the context of the present invention are induced pluripotent stem cells, commonly abbreviated as iPS cells or iPSCs, a type of pluripotent stem cell artificially derived from a non-pluripotent cell, typically an adult somatic cell, by inserting certain genes. Induced pluripotent stem cells are believed to be identical to natural pluripotent stem cells, such as embryonic stem cells in many respects (and therefore encompassed by the term "embryonic stem cells" in the context of the present invention), such as the expression of certain stem cell genes and proteins, chromatin methylation patterns, doubling time, embryoid body formation, teratoma formation, viable chimera formation, and potency and differentiability, but the full extent of their relation to natural pluripotent stem cells is still being assessed. Induced pluripotent stem cells are in general generated by the following method: (1) Isolation and cultivation of donor cells. (2) Transfection of cells with stem cell-associated genes (e.g. colored cells may indicate the cells expressing the exogenous genes). (3) Harvest and cultivation of the cells according to ES cell culture, e.g. using mitotically inactivated feeder cells. (4) Usually, a small subset of the transfected cells becomes iPS cells and generates ES-like colonies.

Transfected genes include the master transcriptional regulators Oct-3/4 (Pouf51) and Sox2, although it is suggested that other genes enhance the efficiency of induction. After 3-4 weeks, small numbers of transfected cells begin to become morphologically and biochemically similar to pluripotent stem cells, and are typically isolated through morphological selection, doubling time, or through a reporter gene and antibiotic infection.

Additionally, the stem cells of the second population may be genetically modified. This may be of particular interest in order to study the function and activity of a particular gene of interest (e.g. during embryonic development) or in order to produce animals with desired properties (e.g. particular productive farm animals) resulting from the presence of a transgene or absence of a particular gene.

For example, knockout mice may be used for loss of function experiments, such as in a gene knockout experiment, in which an organism is engineered to lack the activity of one or more genes. This allows to analyze the defects caused by this mutation, and can be considerably useful in unearthing the function of a gene. A knockout experiment involves the creation and manipulation of a DNA construct *in vitro,* which, in a simple knockout, consists of a copy of the desired gene which has been slightly altered such as to inhibit its function. The construct is then taken up by embryonic stem cells of the second population, where the engineered copy of the gene replaces the organism's own gene. These stem cells may than be used for the methods of the invention.

Alternatively or additionally, gain of function experiments, the logical counterpart of knockouts, may be carried out. These are sometimes performed in conjunction with knockout experiments to more finely establish the function of the desired gene. The process is much the same as that in knockout engineering, except that the construct is designed to increase the function of the gene, usually by providing extra copies of the gene or inducing synthesis of the protein more frequently.

Tracking experiments seek to gain information about the localization and interaction of the desired protein. One way to do this is to replace the wild-type gene with a 'fusion' gene, which is a juxtaposition of the wild-type gene with a reporting element such as Green Fluorescent Protein (GFP) or small sequences which will serve as binding motifs to monoclonal antibodies that will allow easy visualization/detection of the products of the genetic modification. Accordingly, a construct coding for the respective fusion protein may be introduced into the second population of stem cells.

Expression studies aim to discover where and when specific proteins are produced. In these experiments a gene's promoter is reintroduced into an organism with the gene replaced by a reporter gene such as GFP or an enzyme that catalyzes the production of a dye. Thus the time and place where a particular protein is produced can be observed. Expression studies can be taken a step further by altering the promoter to find which pieces are crucial for the proper expression of the gene and are actually bound by transcription factor proteins; this process is known as promoter bashing.

However, the person skilled in the art will understand that the second population may also be modified in a still different manner and/or adapted for a different study design. These modifications are also encompassed by the present invention.

As detailed above, the cells of the first and second population of embryonic stem cells are co-cultivated under suitable conditions for the method of the invention. Suitable conditions include an appropriate temperature and gas mixture (typically, 37°C, 7.5% CO₂) in a cell incubator. Aside from temperature and gas mixture, the most commonly varied factor in culture systems is the growth medium. Recipes for growth media can vary in pH, glucose concentration, growth factors, and the presence of other nutrient components. The factors used to supplement media are often derived from animal blood, such as calf serum or by a feeder layer. Culture conditions may vary from species to species; however, typical conditions are the following are:

DMEM high glucose, 15% FCS, 1x nonessential amino acids, β-mercaptoethanol (0.1 M, fin conc.) and 10000 U/ml Penicillin/Streptomycin. Aggregates are cultivated typically for three days (or until the size of a natural blastocyst is reached and/or signs of cavitation are visible). In general, any media supporting growth of embryonic stem cells might be used, also the amount of FCS might be increased or reduced or even replaced. β-Mercaptoethanol might be omitted, also the nonessential amino acids. Pen/Strep can be omitted as well. The skilled person will be able to select suitable conditions pending on the species and embryonic stem cells used for the method of the invention.

Suitable conditions are also given in the Example.

Preferably, ES^{TSi} and ES Cells are obtained e.g. by trypsinization, and diluted to contain 2:1 ratio of the given cells in DMEM, 15 % FCS, 1 µg/µl 4-hydroxy-tamoxifen. Single droplets of a total volume of 30 µl containing 16 ES^{TSi} and 8 KNUT1-ES cells are applied to a suitable cell culture container (e.g. application onto the surface of an inverted lid of a 15 cm plastic tissue culture dish using an Eppendorf 12 channel pipette (Eppendorf, Hamburg, Germany)). Droplets containing the cells (e.g. 84) are placed on one single tissue culture container such as the above lid. Afterwards, the container may be closed (e.g. the lid may be reverted and put onto a 15 cm tissue culture dish filled with e.g. 12.5 ml PBS) allowing for a humidified atmosphere. Setup may be incubated in a tissue culture incubator with e.g. 7.5 % CO₂ (Heraeus, Germany).

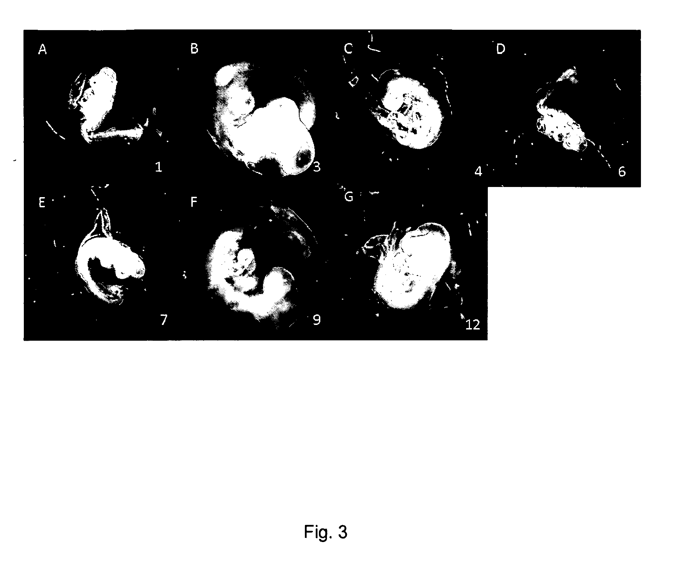

In a particular embodiment of the invention the cells may be grown in a hanging drop system (see e.g. Fig. 3), in which drops including media and cells of both populations are applied to the interior of a lid which is then reverted and placed on a container so as to form a humidified chamber.

In a preferred embodiment of the invention, the embryonic stem cells of the first and second population are mammalian, particularly of the same or closely related animal species, especially of the same or closely related variety. The mammal may be any mammal, however companion and farm animals (such as horse, camel, goat, cow, sheep and pig) and common lab-animals (such as mouse, rat, guinea pig, cat, dog, rabbit and monkey, especially mouse) are preferred.

In still another preferred embodiment of the invention, the total number of cells of the first and second population amounts to about 12 to 48, particularly about 18 to 30, especially about 24 at the beginning of the co-cultivation. The term "about" is used to allow a certain variance above or below the indicated value in order to make allowance for measurement inaccuracies and/or variations inherent to the methods. Preferably, the term about allows for a 20 % variance, more preferably a 10 % variance, even more preferably a 5 % variance.

In still another preferred embodiment of the invention the ratio of first and second embryonic stem cells at the beginning of the co-cultivation is in the range of from 1:1 to 4:1, particularly in the range of from 1.5:1 to 3:1, especially about 2:1. The term "about" is as defined above.

A second aspect of the invention relates to an induced blastocyst-like structure producible according to any of the methods detailed above. The induced blastocyst-like structure is as defined above.

In a third aspect of the invention the blastocyst may be used for the production of a (non-human) embryo or a (non-human) animal. Suitable methods for producing the same are detailed below.

A further aspect of the present invention relates to a method of producing a (non-human) embryo comprising
a) cultivating an induced blastocyst-like structure under conditions conducive to the production of an embryo thereby producing an embryo.

The cultivation of the blastocyst-like structure may be continued to obtain an embryo, wherein the conditions may be as detailed above in the context of the co-cultivation of stem cells of the first and second population.

A still further aspect of the present invention relates to a method of producing a (non-human) embryo or (non-human) animal comprising
a) cultivating an (non-human) induced blastocyst-like structure under conditions conducive to the production of an embryo thereby producing an embryo,
b) transferring the embryo into a recipient animal to establish pregnancy, and
c) isolating the (non-human) embryo or (non-human) animal from the recipient.

Step a) of the method may be carried out as described above in connection with the method for producing a (non-human) embryo. Thereafter, the embryo may be transferred into a recipient animal, in general a surrogate mother, in order to establish pregnancy in that recipient.

For embryo transfer one or several embryos may be placed into the uterus of the female with the intent to establish a pregnancy. The embryo can be either "fresh" or "frozen" that is it has been generated in at a prior date, cryopreserved, and is thawed just prior to the transfer. The uterine lining (endometrial) needs to be appropriately prepared so that the embryo(s) can implant. In general, the recipient is treated with hormones (such as estrogen and progesterone) in order to provide for a lining appropriately developed in relation to the status of the embryo.

For embryo transfer may be carried out as follows: The procedure of embryo transfer starts by placing a speculum in the vagina to visualize the cervix, which is cleansed with saline solution or culture media. A transfer catheter is loaded with the embryos. The catheter is inserted through the cervical canal and advanced into the uterine cavity where the embryos are deposited. The catheter is then withdrawn. An abdominal ultrasound may be used to ensure correct placement. After embryo transfer it may be necessary to keep the recipient on estrogen and progesterone medication; pregnancy testing may be done for example typically two weeks after the transfer. Depending on the experimenter's interest the pregnancy may be either interrupted (and the resulting embryo may be studied) or for the producing of a fully developed animal the pregnancy may be proceeded until the respective gestation period of the animal. The fully developed animal may be isolated by natural birth or by artificial removal such as sacrificing the recipient and opening of the abdominal cavity or cesarean.

Alternatively, the animal may be anaesthetized and the skin may be opened on the dorsal midline using fine scissors. The Uterus may be located and pulled out by grabbing the fat pad surrounding the ovary. Using a hypodermic needle, a hole may be punched at the junction of uterus and oviduct. Afterwards e.g. a mouth-controlled glass pipette loaded with the iBLASTs may be inserted and the iBLASTs can be transferred into the uterus.

A suitable method exemplified on mice is also detailed in the Example.

Another aspect of the present invention relates to a kit suitable for the production induced blastocyst-like structure, a (non-human) embryo or (non-human) animal, the kit comprising
- a first population of embryonic stem cells modified to allow for differentiation into trophectoderm,
- optionally a second population of embryonic stem cells, and
- optionally media and/or instructions for the co-cultivation of the first and second population of cells.

The first population of embryonic stem cells modified to allow for differentiation into trophectoderm, a second population of embryonic stem cells and the media, if present, may be as defined above or as defined in the Example. The instructions may reflect the methods and uses described herein. The kit may also comprise consumables in order to carry out the method and uses such as suitable plastic material, e.g. culture dishes etc. The culture dish may be adapted to the hanging drop method described above.

In another aspect the invention provides the use of an embryonic stem cell modified to allow for differentiation into trophectoderm, optionally further characterized as defined in any of claims 2 to 4, for the production of induced blastocyst-like structure, a (non-human) embryo or a (non-human) animal. For this, nay of the above methods may be used.

The following Figures and Example are intended to illustrate the invention described herein. However, they are not intended to limit the scope of the claimed invention.

### FIGURES

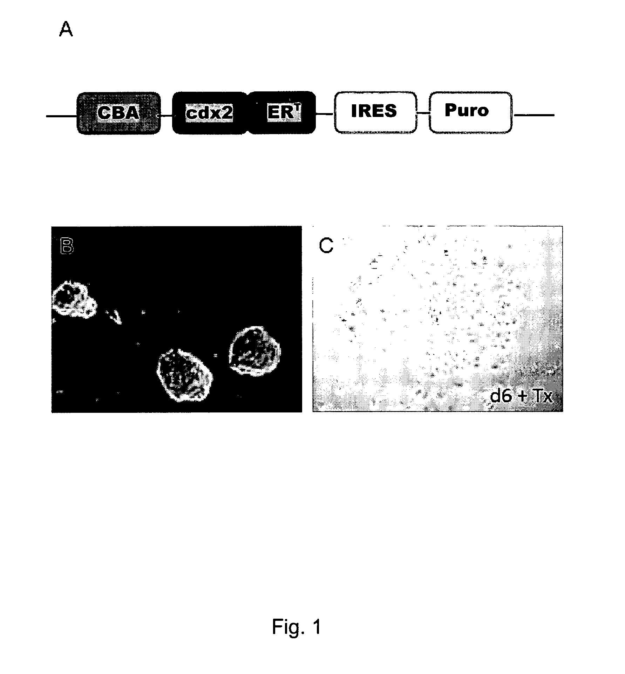

**Figure 1** shows generation of ES^{TSi} cells
   (A) Vector used for the transfection. CBA-chicken beta actin promoter, cdx-2Ek^{T}: fusion of cdx-2 and ER^{T}, IRES: internal ribosomal entry site, puro: puromycin resistance cassette (see also Niwa, H. et al., Cell 123, 917-29 (2005)), (B) ES^{TSi} Cells after 6 days in culture without 4-Hydroxytamoxifen, (C) ES^{TSi} Cells after 6 days with 4-Hydroxytamoxifen.
**Figure 2** shows the "Hanging Drop System"
   Droplets of 30 µl were placed on the surface of an inverted lid of a 15 cm tissue culture dish. Lid was reverted and placed onto the dish which contains 12.5 ml of PBS in order to generate a humidified chamber for incubation.
**Figure 3** shows embryos obtained from fosters at day 10.5 dpc
   (A-G) show stereomicroscopic photographs of the embryos. Pictures taken at identical magnification (50 x) to demonstrate size difference.
**Figure 4** confirms the iBLAST origin of the embryos
   (A) Schematic of the transgene used to induce TS differentiation in ES^{TSi} Cells. Arrows in (A) indicate annealing sequences of the primers used. (B) PCR of DNA generated from the placentae of the iBLAST transfer. Numbers refer to the respective embryos; arrow indicates the expected band size. PK: Positive Control (ES^{TSi} Cells); NK1, NK2: Negative Controls; H₂O: Primer only; M: Size Marker.

### EXAMPLE

### EXAMPLE 1: In vitro generation and sucessful in utero embryogenesis of blastocyst like structures

### Material and Methods:

*ES Cells:* SB1 - ES Cells were derived by us from animals 75 % 129/sv; 25% C57/B16 and kept under standard culture conditions (cf. Schorle, H. et al., Nature 352, 621-4 (1991)). KNUT1 ES cells are derived by us using F1 C57/B16x129sv animals. SB1 and KNUT are grown on a feeder layer of mitomycin C treated or irradiated fibroblasts in DMEM high glucose, 15% FCS, 1000 U/ml, ESGRO (LIF), 1 x nonessential amino acids, β-mercaptoethanol and 10000 U/ml Penicillin/Streptomycin. Cells are monitored daily and trypsinzed every 2^{nd} or 3^{rd} day. Using this regimen, cells grow in typical tight packed colonies on top of the feeder layer showing little to no sign of differentiation. Cells used in the experiment were passage 10-12.

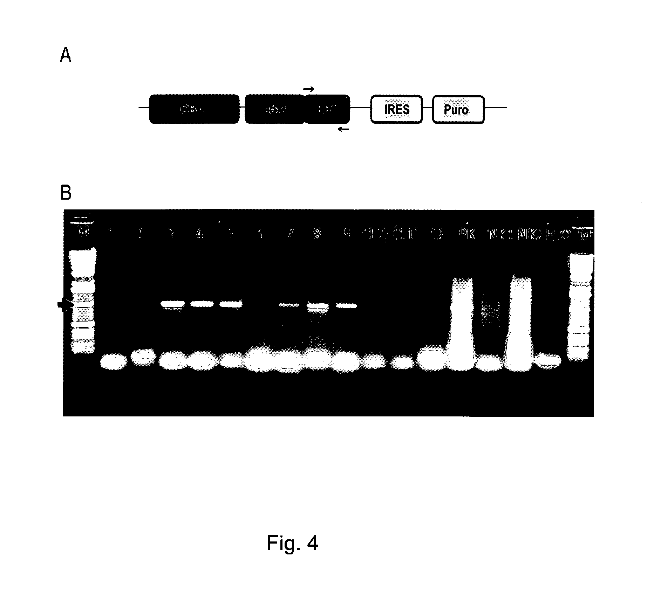

*Transfection:* 1x10e6 SB1 ES cells were electroporated with the inducible Cdx-2 expression cassette (Niwa et al., supra; see also Figures 1A and 4A), clones were selected using puromycin (1.4 µg/µl). Chicken beta actin promoter is placed 5' to the cDNA consisting of a fusion of murine cdx-2 and mutated ligand binding domain of estrogen receptor (ER^{T}). 3' of this cassette, an internal ribosomal entry site (IRES) is placed followed by a puromycin resistance cassette (Puro). Using this construct results in a polycistronic messenger RNA encompassing cdx2ER IRES and puro. CBA is active in ES and TS cells, the fusion cdx2/ERT is being transcribed and translated into a protein but inactive until 4-hydroxytamoxifen is added. IRES results in reinitiation of ribosomes 3' of the cdx2ERT and hence the production of protein from the puro cassette mediating the resistance to this antibiotic. The resistance cassette was used to find the ES clones which had stably integrated the construct in their genomic DNA.

*TS induction:* Selected clones were grown with 1 µg/µl 4-hydroxy-tamoxifen to induce Cdx-2 activity. Cultures were scored for changes in morphology.

*Hanging drop culture:* ES^{TSi} and ES Cells were trypsinized and diluted to contain 2:1 ratio of the given cells in DMEM, 15 % FCS, 1 µg/µl 4-hydroxy-tamoxifen. Single droplets of a total volume of 30 µl containing 16 ES^{TSi} and 8 KNUT1-ES cells were applied onto the surface of an inverted lid of a 15 cm plastic tissue culture dish using an Eppendorf 12 channel pipette (Eppendorf, Hamburg, Germany). 84 droplets were placed on one single tissue culture lid. Afterwards the lid was reverted and put onto a 15 cm tissue culture dish filled with 12.5 ml PBS allowing for a humidified atmosphere. Setup was incubated in a tissue culture incubator with 7.5 % CO₂ (Heraeus, Germany).

*Uterine transfer of iBLAST:* Pseudo pregnant females were produced by timed matings using vasectomised male studs following standard procedures (Hogan, B., Beddington, R. & Constantini, F. Manipulating the Mouse Embryo (Cold Spring Harbor Laboratory Press, 1994)). Day of plug was considered day 0.5 of pregnancy (dpc). On day 2.5 dpc the female was anaesthetised and 10 iBLAST were transferred per uterine horn using a fine drawn mouth-controlled Pasteur pipette following standard procedures (Hogan et al., supra).

*DNA Preparation and PCR Analysis:* DNA was generated from labyrinthine layer of the placenta containing derivates of the embryonal trophectoderm cells using standard protocol (Werling, U. & Schorle, H, Genesis 32, 127-9 (2002)). PCR specific for the Cdx-2 ER^{T} transgene was applied using the following primers:
5'ERT: GGATCCCGAATTGAAATGGGTGCT (SEQ ID NO: 1)
3'ERT: GGATCCTCAGATCGTGTTGG GGAAGCC (SEQ ID NO: 2)

The resulting product was 961 bp in size. PCR profile: after initial 94 °C for 2 min, profile 94 °C for 30 s, 61 °C for 30 s, 72 °C for 50 s, was run 35x times, products were separated on a 1% agarose gel and photographed using an Intas Gel Documentation System (Intas, Cologne, Germany).

### Results:

*Generation of ES^{TSi} Cells:* SB 1 ES Cells were transfected with a tamoxifen-inducible Cdx-2 expression construct (Fig. 1, A) (Niwa, supra). Clones were established and their ability to differentiate into TS cells was tested in cell culture (Fig.1., B compare to C). The clone used confirmed the results published by Niwa et al., (vide supra) and was named ES^{TSi} (for **ES** cells, **T**rophectoderm **S**tem Cell **i**nducible) and used for following studies.

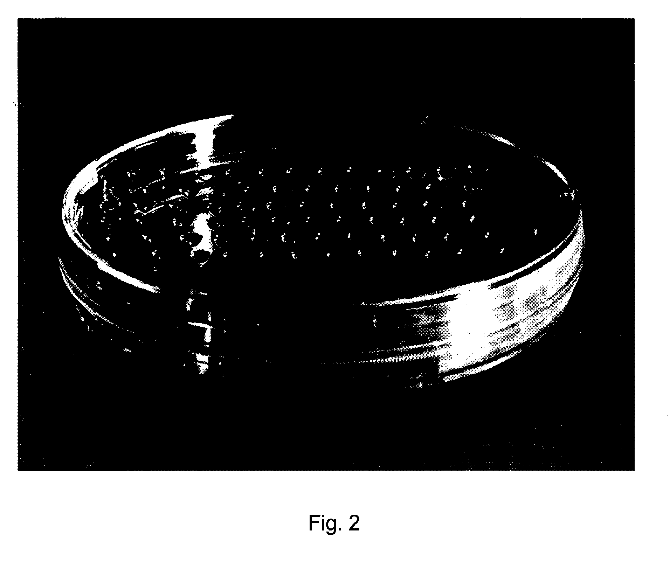

*Generation of iBLASTs:* To avoid attachment, adherence, and spreading of the cells on a tissue culture surface, we decided to incubate the cells using a hanging drop culture system depicted in Figure 2. Prior to the experiment, ES and ES^{TSi} cells were trypsinized, harvested, and counted. To generate blastocyst-like structures, we aimed to setup a co-culture of wildtype KNUT1-ES cells (derived in our group) and ES^{TSi} cells. Cultures were monitored daily and the appearance of cellular aggregates was observed. Initial experiments using ratios of 1:1, 2:1 and 4:1 of ES^{TSi} cells and ES cells showed that after 5 days of incubation a ratio 2:1 produced the most homogeneous cellular aggregates which were comparable in size to a regular blastocyst. Using antibodies we were able to show that these structures had Cdx-2 -positive cells lining the outside, while Oct4-positive cells were detected on the inside. Further, blastocyst-like cavitation could be observed in these structures. So, based on size, expression of marker genes, distribution of the Cdx-2-positive, and Oct4-positive cells we conclude that using this protocol we had induced **Bla**stocyst-like structures (iBLAST). In general, approximately 20 % of the cellular aggregates succeed in forming iBLASTs.

*Transfer in UterilTest for embryonic development:* Next, we tested whether the iBLASTs generated would be able to develop into embryos when transferred into a pseudo pregnant female. Here, we decided to use day 2.5 pseudo pregnant female animals since this timing has been used successful with traditional blastocyst transfer. iBLASTs were initiated, incubated for 3 days and transferred into the uterus of pseudo pregnant females. To check for proceeding embryogenesis, fosters were sacrificed at day 10.5 of pregnancy. While 5 fosters had no implantation sites, foster number 6 showed 10 implantations, containing seven embryos (Fig. 3). Here a difference within the group of embryos collected was apparent. While one embryo (Fig. 3, B) represented correct developmental age and size the others displayed a delay of one (Fig. 3, C, F, G) or even two developmental days (Fig.3, A, D, E). This result shows that the iBLASTs generated can support full developmental potential up to day 10.5 of development.

*Confirmation of the iBLAST Origin of the recovered embryos:* We hypothesize that the ES^{TSi} cells contribute to the development of the trophectoderm lineage while the ES cells form the embryo proper. Since the ES^{TSi} cells harbour the inducible Cdx-2 transgene, we setup a PCR analysis using DNA prepared from the placentas of the developing embryos of the iBLAST culture. As shown in Fig. 4 DNA derived from the placentas of implantation # 1, 3, 4, 5, 6, 7, 8 and 9 show the characteristic band, confirming the iBLAST origin. Implantation site #2 and #5 was empty containing no embryo; DNA derived from unidentified cellular aggregates (Fig. 4, # (10), # (11)) failed to produce any band. Interestingly, DNA from #12 was negative for the transgene while giving a reasonable embryo (compare to Fig.3, G). Since we cannot formally exclude a mistake in DNA isolation on # 2 and # 12 this result might represent a technical problem which will be overcome while repeating the experiment.

In summary, we show that Blastocyst-like structures (iBLAST) can be generated using a genetically modified ES cell line (ES^{TSi}) which induces trophectoderm differentiation. After incubation of ES^{TSi} cells and regular ES cells iBLASTs are formed which express the markers of pluripotency Oct4 and Cdx2 in a manner similar to blastocysts. When transplanted into uteri of pseudopregnant mice, the iBLASTs implant to give rise to embryos. The iBLAST origin of the embryos was confirmed by using a PCR specific for the Cdx-2 ER transgene.

## Claims

1. A method of producing an induced blastocyst-like structure comprising the steps of:
a) co-cultivating a first population of embryonic stem cells modified to allow for differentiation into trophectoderm with a second population of embryonic stem cells under conditions conducive to the production of an induced blastocyst-like structure thereby producing an induced blastocyst-like structure.

2. The method of claim 1, wherein the embryonic stem cells of the first population are genetically modified, particularly genetically modified to include a caudal related homeobox-2 (cdx-2) gene, a transcription factor activating enhancer binding protein 2 gamma (TFAP2g) gene and/or a TEA domain family member 4 (TEAD4) gene, particularly a cdx-2 gene.

3. The method of claim 2, wherein the expression of the gene(s) is inducible.

4. The method of claim 3, wherein the gene is under the control of an inducible promoter or a controllable ligand binding domain of a nuclear hormone receptor (LBD) or a functionally active variant thereof, particularly a ligand binding domain of the estrogen receptor or functionally active variant thereof.

5. The method of claim 3 or 4, wherein the expression is induced during and/or before co-cultivation of the embryonic stem cells of the first and second population.

6. The method of any of claims 1 to 5, wherein the embryonic stem cells of the second population are pluripotent.

7. The method of any of claims 1 to 6, wherein embryonic stem cells of the first and second population are mammalian, particularly of the same or closely related animal species, especially of the same or closely related variety.

8. The method of any of claims 1 to 7, wherein at the beginning of the co-cultivation the total number of cells of the first and second population amounts to about 12 to 48, particularly about 18 to 30, especially about 24.

9. The method of any of claims 1 to 8, wherein the ratio of first and second embryonic stem cells at the beginning of the co-cultivation is in the range of from 1:1 to 4:1, particularly in the range of from 1.5:1 to 3:1, especially about 2:1.

10. An induced blastocyst-like structure producible according to a method of any of claims 1 to 9.

11. Use of an induced blastocyst-like structure according to claim 10 for the generation of a non-human embryo or a non-human animal.

12. A method of producing a non-human embryo comprising
a) cultivating an induced blastocyst-like structure under conditions conducive to the production of an embryo thereby producing an embryo.

13. A method of producing a non-human animal comprising,
a) cultivating an non-human induced blastocyst-like structure under conditions conducive to the production of an embryo thereby producing an embryo,
b) transferring the embryo into a recipient animal to establish pregnancy, and
c) isolating the non-human animal from the recipient.

14. A kit suitable for the production induced blastocyst-like structure, a non-human embryo or non-human animal, the kit comprising
- a first population of embryonic stem cells modified to allow for differentiation into trophectoderm,
- optionally a second population of embryonic stem cells, and
- optionally media and/or instructions for the co-cultivation of the first and second population of cells.

15. Use of an embryonic stem cell modified to allow for differentiation into trophectoderm, optionally further **characterized** as defined in any of claims 2 to 4, for the production of induced blastocyst-like structure, a non-human embryo or a non-human animal.
